(19) Europäisches Patentamt
European Patent Office
Office européen des brevets

(11) Publication number: **0 180 993 B1**

(12) **EUROPEAN PATENT SPECIFICATION**

(45) Date of publication of patent specification: **27.02.91**

(51) Int. Cl.5: **C07D 249/20**

(21) Application number: **85114203.4**

(22) Date of filing: **07.11.85**

The file contains technical information submitted after the application was filed and not included in this specification

(54) **Process for preparing 2,2'-methylene-bis-(4-hydrocarbyl-6-benzo-triazolylphenols).**

(30) Priority: **09.11.84 JP 236290/84**

(43) Date of publication of application:
**14.05.86 Bulletin 86/20**

(45) Publication of the grant of the patent:
**27.02.91 Bulletin 91/09**

(84) Designated Contracting States:
**BE CH DE FR GB LI NL**

(56) References cited:
**EP-A- 0 180 991**
**EP-A- 0 180 992**
**DE-A- 1 670 951**

**CHEMICAL ABSTRACTS, vol. 83, no. 21, 24 Nov 1975, Columbus, OH (US); O.H.HISHMAT et al.: "Alkylaminomethylation and chloromethylation of 2,3-diphenyl hydroxybenzofurans", p. 539, no. 178672x**

**CHEMICAL ABSTRACTS, vol. 77, no. 10, Sep 1972, Columbus, OH (US); p.28, no.62720h**

(73) Proprietor: **ADEKA ARGUS CHEMICAL CO., Ltd.**
**Yoko Bldg. 1-4-5 Hongo Bunkyo-Ku**
**Tokyo(JP)**

(72) Inventor: **Kubota, Naohiro**
**Ageohigashi-danchi 3-105 404-1 Ageomura**
**Aego City Saitama(JP)**
Inventor: **Nishimura, Atsushi**
**4-19-3 Washinomiya Washinomiya**
**Saitama(JP)**

(74) Representative: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**D-8000 München 40(DE)**

**EP 0 180 993 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

2,2'-Methylene-bis-(4-hydrocarbyl-6-benzotriazolyl-phenols) are known light stabilizers for plastics, and a process for preparing them is disclosed in Chemical Abstracts 74 53666f (1971) and 77 62720h (1972).

U.S. patent No. 3,936,305, patented February 3, 1976, to Hiraishi, Futaki, Horii and Yamashita, discloses a group of alkylidene-bis-benzotriazolyl phenols having the formula:

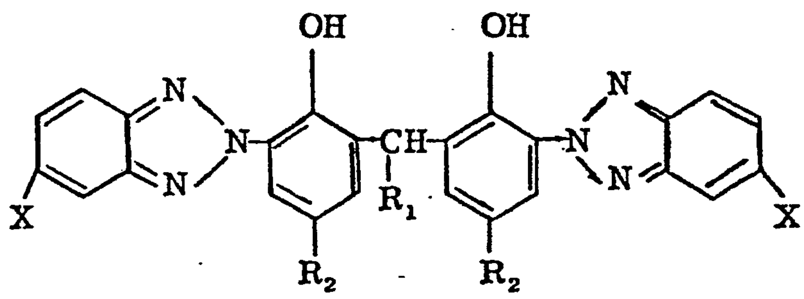

wherein $R_1$ is an alkyl group having 1 to 13, preferably 5 to 13 carbon atoms, $R_2$ is an alkyl group having 1 to 18 carbon atoms and X is hydrogen, a halogen, an alkyl, an alkoxy, an aryloxy, an aralkyloxy or an aryl group.

These compounds are prepared by a synthesis illustrated for 2, 2'-octylidene-bis(4-methyl-6-(5"-methylbenzotriazolyl)-phenol)).

Dried hydrogen chloride is introduced into a benzene solution containing 129.6 g of p-cresol, 64.1 g of n-caprylaldehyde and 2 ml of n-dodecylmercaptan at room temperature for 4 hours. This is left to stand overnight at room temperature. Thereafter, the reaction liquid is washed with water, washed with IN aqueous solution of sodium bicarbonate until the wash liquid shows weak

These compounds are prepared by a synthesis illustrated for 2,2'-octylidene-bis(4-methyl-6-(5"-methylbenzotriazolyl)-phenol)).

Dried hydrogen chloride is introduced into a benzene solution containing 129.6 g of p-cresol, 64.1 g of n-caprylaldehyde and 2 ml of n-dodecylmercaptan at room temperature for 4 hours. This is left to stand overnight at room temperature. Thereafter, the reaction liquid is washed with water, washed with IN aqueous solution of sodium bicarbonate until the wash liquid shows weak acidicity and then again washed with water. After the benzene layer is dried, the solvent is distilled out and the residue is distilled under reduced pressure to obtain a fraction of 165°-175° C/l Torr. The fraction is recrystallized from n-hexane to obtain white crystal of 2,2'-octylidene bis(4-methylphenol) having a melting point of 107.8°-108.8° C.

Separately, 120 ml of concentrated hydrochloric acid and 40 ml of water are added to 60.8 g of 4-methyl-2-nitroaniline and these are sufficiently sitrred. Thereafter, 50 ml of water containing 28.9 g of sodium nitrite is added dropwise thereto at 0° C over a period of 10 minutes. Furthermore, this is stirred for another 100 minutes and a small amount of sulfamic acid is added thereto and undissolved matter is filtered off to obtain a diazonium solution.

32.6 g of 2, 2'-octylidene bis(4-methylphenol) obtained hereinabove is dissolved in a mixed solvent of 300 ml of methyl alcohol and 200 ml of acetone in which 50 g of sodium hydroxide is dissolved. To this solution is added dropwise said diazonium solution with stirring at a temperature of 0°-10° C. After stirring for another 2 hours, glacial acetic acid is added thereto to obtain a pH of 4. Thus obtained oily product is recrystallized from ethanol.

6.5 g of this 2,2'-octylidene bis(4-methyl-6-(4"-methyl-2"-nitrophenylazo) phenol) is suspended in 100 ml of ethanol and heated to reflux temperature. 50 ml of water containing 8.4 g of sodium hydroxide is added to the suspension and furthermore, 6.5 g of zinc powder is added little by little. Refluxing is further carried out for 1 hour. Thereafter, the zinc powder is filtered out with warm and pH of the filtrate is made 4-5 with IN hydrochloric acid to separate crystal, which is filtered off. The crystal is recrystallized from ethanol to obtain the 2,2-octylidene-bis-(4-methyl-6-(5"-methyl benzotriazolyl) phenol).

Chemical Abstracts 74 53666f (1971) discloses the preparation of methylene bis-(benzotriazolyl phenols) by reacting 2,2'-methylene-bis-phenol with an o-nitrodiazonium salt, followed by hydrogenating the resultant azo compound. However, the yield is rather low, and the product contains a monosubstituted impurity.

Chemical Abstracts 77 62720h (1972) discloses the preparation of methylene-bis-(benzotriazolyl phenols) by reacting benzotriazolyl phenol with formaldehyde in sulfuric acid. However, the yield in this

method is also low, and the reaction conditions are impractical.

In accordance with this invention, a process is provided which enables the preparation of 2,2'-methylene-bis-(4-hydrocarbyl-6-benzotriazolyl-phenols) under mild conditions in high yield.

A 4-hydrocarbyl-6-benzotriazolyl-phenol of formula I is reacted with the corresponding amine $HNR_1R_2$ and formaldehyde in an organic solvent to produce a Mannich base of formula II. The Mannich base is reacted with itself or a 4-hydrocarbyl-6-benzotriazolyl-phenol of formula I, thereby forming the desired 2,2'-methylene-bis-(4-hydrocarbyl-6-benzotriazolyl phenol.

The following is an outline of the reactions in this synthesis:

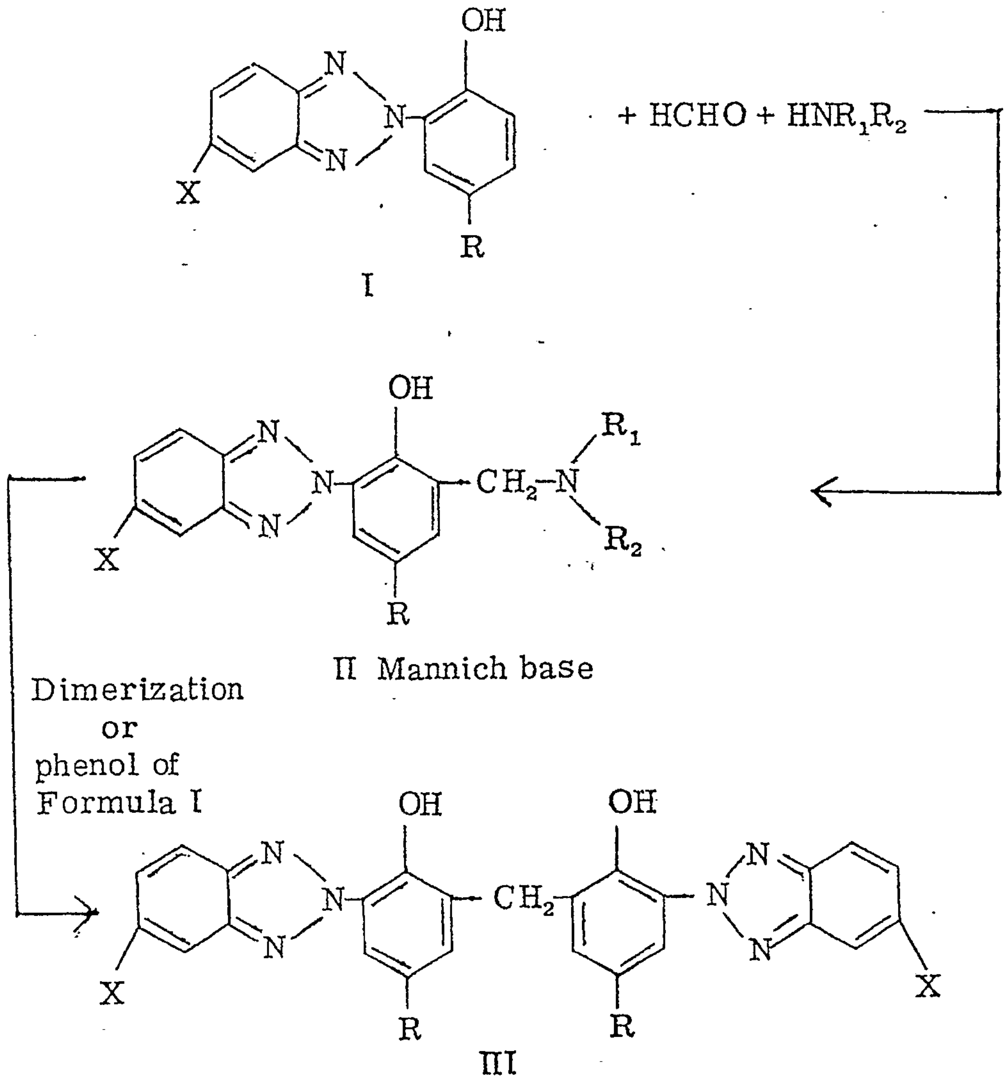

I
II Mannich base
III

In the above formulae, R is selected from the group consisting of alkyl having from one to twelve carbon atoms; arylalkyl having from seven to fourteen carbon atoms and cycloalkyl having from three to twelve carbon atoms;

X is selected from the group consisting of hydrogen, halogen, alkyl having from one to twelve carbon atoms, aryl having from six to ten carbon atoms, arylalkyl having from seven to sixteen carbon atoms, alkoxy having from one to twelve carbon atoms, aryloxy having from six to ten carbon atoms; and arylalkoxy having from seven to sixteen carbon atoms;

$R_1$ and $R_2$ are selected from the group consisting of hydrogen; alkyl having from one to six carbon atoms, and $R_1$ and $R_2$ taken together to form a four to six member heterocyclic ring including a nitrogen atom; provided, that at least one of $R_1$ and $R_2$ is not hydrogen.

The invention accordingly provides a process for preparing alkylidene-bis-benzotriazolyl phenols of Formula III, comprising

(1) reacting a 4-hydrocarbyl-6-benzotriazolyl phenol having the formula I:

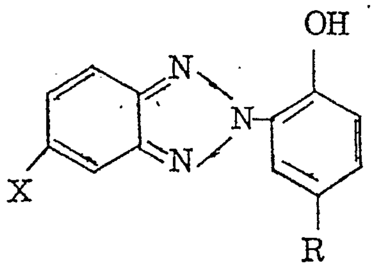

I

with an amine $HNR_1R_2$ and formaldehyde in an organic solvent to produce a Mannich base having formula II:

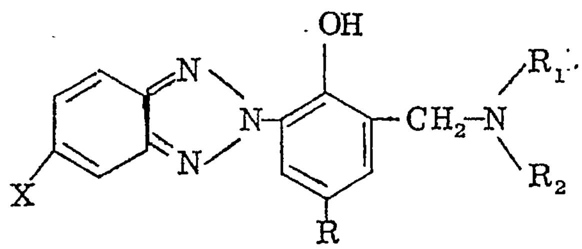

II; and

(2) reacting the Mannich base with itself or a 4-hydrocarbyl-6-benzotriazolyl phenol having fromula I, thereby forming a 2, 2'-methylene-bis-(4-hydrocarbyl-6-benzotriazolyl-phenol of formula III.

Exemplary R alkyl include methyl, ethyl, propyl, isopropyl, butyl, isobutyl, t-butyl, amyl, isoamyl, hexyl, heptyl, octyl, isooctyl, 2-ethylhexyl, 1,2,3,3-tetramethylbutyl, nonyl, decyl, dodecyl;

Exemplary R arylalkyl include benzyl, α-methylbenzyl, cumyl, phenethyl, phenpropyl, phenbutyl, phenoctyl, trimethyl benzyl, butylbenzyl, hexylbenzyl, dibutylbenzyl;

Exemplary cycloalkyl include cyclopropyl, cyclobutyl, cyclopentyl, cyclohexyl, cycloheptyl, cyclooctyl, and cyclododecyl;

Exemplary X halogen include fluorine, chlorine and bromine, exemplary X alkyl include methyl, ethyl, propyl, isobutyl, isoamyl, and hexyl; exemplary X alkoxy include methoxy, ethoxy, butoxy and octoxy;

Exemplary amine $HNR_1R_2$ include primary alkyl amines such as monomethylamine, monoethylamine, monopropylamine and monobutylamine; secondary alkyl amines such as dimethylamine, diethylamine, di-n-propylamine, diisopropylamine, di-n-butylamine, di-t-butylamine, diisobutylamine, diamylamine, ethyl-methylamine and ethyl-isopropylamine; and heterocyclic amines such as morpholine, piperidine and pyrrolidine.

Formaldehyde as such or in any of its polymeric forms can be used in the process this invention, such as gaseous formaldehyde; aqueous solutions of formaldehyde; paraformaldehyde; trioxane; trioxymethylene; tetraoxymethylene; and other solid polymers of formaldehyde.

Any inert organic solvent that is a solvent for the reactants can be used in this invention, including alkanols such as methanol, ethanol, isopropanol and n-butanol; hydrocarbons such as hexane, heptane, benzene, toluene, xylene, petroleum ethers, and mineral spirits; and cycloaliphatic ethers such as furan, tetrahydrofuran and dioxane. The amount of solvent is not critical, and can suitably be within the range from about 50 to about 500 weight % based on the amount of benzotriazolyl phenol present.

In the reaction of 4-hydrocarbyl-6-benzotriazolyl-phenol of formula (I) with amine and formaldehyde in the organic solvent, the amounts of amine and formaldehyde can be stoichiometric, or slightly less, and preferably each is within the range from about 0.5 to about 1 mole, per mole of 4-hydrocarbyl-benzotriazolyl phenol.

The reaction of Mannich base with itself or 4-hydrocarbyl-6-benzotriazolyl-phenol to produce 2,2'-methylenebis-(4-hydro-carbyl-6-benzotriazolyl phenol) is preferably carried out in the presence of an alkaline catalyst. Examples of alkaline catalysts are lower alkali metal alcoholates, such as sodium methylate and sodium ethylate; alkali metal hydroxides, such as sodium hydroxide and potassium hydroxide; and alkali metal alkaline salts, such as potassium carbonate and sodium carbonate. The amount of the alkaline catalyst is not critical, and small amounts are usually sufficient. A preferred amount is within the range from 0.001 to 50 parts by weight, preferably from 0.01 to 8 parts by weight, per 100 parts of Mannich base.

The reactions can be carried out over a wide range of temperatures. A suggested range that is

satisfactory in most cases is from about 20° C to about 200° C, preferably from about 30° C to about 150° C

The following Examples represent preferred embodiments of the process of the invention.

Example 1

Preparation of 2,2'-methylene-bis-(4-methyl-6-benzotriazolyl-phenol)

4-Methyl-6-benzotriazolyl-phenol 225 g, diethylamine 110 g, and paraformaldehyde 51.8 g were dissolved in 250 ml of butanol, and heated with stirring at reflux temperature (95° C to 105° C) for 24 hours. The solvent was vacuum distilled off, and 308 g of 2-diethylaminomethyl-4-methyl-6-benzotriazolyl-phenol Mannich base was obtained as residue. (Yield = 99%)

This Mannich base, 7.8 g, was dissolved in 20 ml of xylene, and sodium methylate (28% methanol solution) 0.15 g was added. The solution was heated with stirring under reflux at 140° C to 150° C for 10 hours while a nitrogen stream was passed through the reaction mixture. The solvent was vacuum distilled off, and 6.1 g of crude product was obtained as residue. (Purity = 91%; Yield = 96%)

The crude product was recrystallized from xylene, and a pale yellow powder, 2,2'-methylene-bis-(4-methyl-6-benzotriazolyl-phenol melting at 285° C was obtained.

Example 2

Preparation of 2,2'-methylene-bis-(4-methyl-6-benzotriazolyl-phenol)

6.2 g of the Mannich base obtained in Example 1 and 4-methyl-6-benzotriazolyl-phenol 4.5 g were dissolved in 200 ml of xylene, and sodium methylate (28% methanol solution) 0.2 g was added. The solution was heated with stirring under reflux at 140-50° C for 10 hours with a stream of nitrogen. After distilling of the solvent, and recrystallizing the residue from xylene, the desired product 2,2'-methylene-bis-(4-methyl-6-benzotriazolyl-phenol) was obtained In 95% yield.

Example 3

Preparation of 2,2'-methylene-bis-(4-(1,1,3,3-tetramehtyl) butyl-6-benzotriazolyl-phenol)

2-Diethylaminomethyl-4-(1,1,3,3-tetramethyl)butyl-6-benzotriazolyl-phenol Mannich base was prepared using 4-(1,1,3,3-tetramethyl) butyl-6-benzotriazolyl-phenol by the same procedure as in Example 1. This Mannich base 37 g and 4-(1,1,3,3-tetramethyl) butyl-6-benzotriazolyl-phenol 25 g were dissolved in 60 ml of xylene, and sodium methylate (28% methanol solution) 3.1. g was added. The solution was heated with stirring under reflux at 140-50° C for 10 hours with a stream of nitrogen. After distilling of the solvent, and recrystallizing the residue from xylene, 55.2 g of the crude product was obtained. (Purity = 93%; Yield = 93%)

The crude product was recrystallized from n-heptane, and a white powder, 2,2'-methylene-bis-4-(1,1,3,3-tetramethyl)butyl-6-benzotriazolyl-phenol, melting at 200° C was obtained.

Example 4

Preparation of 2,2'-methylene-bis-(4-cumyl-6-benzotriazolyl-phenol)

2-Diethylaminomethyl-4-cumyl-6-benzotriazolyl-phenol Mannich base was prepared using 4-cumyl-6-benzotriazolyl-phenol by the same procedure as in Example 1. This Mannich base 10.0 g and 4-cumyl-6-benzotriazolyl-phenol 6.6 g were dissolved in 60 ml of xylene, and sodium methylate (28% methanol solution) 3.1 g was added. A white crystalline product, 2,2'-methylene-bis-(4-cumyl-6-benzotriazolyl-phenol)

melting at 190 °C was obtained. (Yield 93%).

Example 5

Preparation of 2,2'-methylene-bis-(4-methyl-6-benzotriazolyl-phenol)

31.0 g of the Mannich base obtained in Example 1 and methyl iodide 30 g were dissolved in 100 g of ethanol, and heated with stirring at reflux temperature (60 °C to 75 °C) for 24 hours. The solvent was distilled off, and the pale yellow crystalline product (methyl-diethyl-2-hydroxy-3-benzotriazolyl-5-methylbenzylammonium iodide) was obtained by recrystallization from ethanol.

This product 9.0 g and sodium methylate (28% methanol solution) 4.0 g were dissolved in 40 g of butoxy ethoxy ethanol, and heated with stirring at reflux temperature (160 °C to 170 °C) for 10 hours while a nitrogen stream was passed through the reaction mixture. The solvent was vacuum distilled off, and 6.1 g of crude product was obtained as residue. (Purity = 91%; Yield = 96%)

The crude product was recrystallized from xylene and the desired product, 2,2'-methylene-bis-(4-methyl-6-benzotriazolyl-phenol) was obtained in 95% yield.

The excellent yields obtained in the above Examples are to be contrasted with the yields when the process of Chemical Abstracts 77 62720h (1972) is used.

Comparative Example A

Preparation of 2,2'-methylene-bis-(4-methyl-6-benzotriazolyl-phenol)

4-Methyl-6-benzotriazolyl-phenol 22.5 g, paraformaldehyde 2.5 g and conc-sulfuric acid 0.5 g were dissolved in 50 ml xylene, and heated with stirring at reflux temperature (140 °C to 150 °C) for 20 hours. The solution was washed with water. When the solvent was distilled off, 22.8 g of a dark brown crude product was obtained.

The crude product contained only 18% of the desired product, 2,2'-methylene-bis-(4-methyl-8-benzotriazolyl-phenol), and most of the crude product was unreacted 4-methyl-6-benzotriazolyl-phenol.

Comparative Example B

Preparation of 2,2'-methylene-bis-(4-(1,1,3,3-tetramethyl)butyl-6-benzotriazolyl-phenol)

4-(1,1,3,3-Tetramethylbutyl)-6-benzotriazolyl-phenol 32.3 g and paraformaldehyde 2.5 g were dissolved in 100 ml of methylene chloride, and 50 g of 80% sulfuric acid was added dropwise over one hour. The solution was stirred at room temperature for 12 hours. The sulfuric layer was separated, and the methylene-chloride layer 25 washed with water and dried. The solvent was distilled off and 22.5 g of brown crude product was obtained.

The crude product was consisting of 4% of unreacted 4-(1,1,3,3-tetramethyl) butyl-6-benzotriazolyl-phenol, 31% of desired product, 2,2'-methylene-bis(4-(1,1,3,3-tetramethyl) butyl-6-benzotriazolyl-phenol), methylenbis(benzotriazolyl-phenol) compounds free from one or two 1,1,3,3-tetramethylbutyl groups at the 4-position (dealkylation products) 52%, and 13% of other unknown compounds.

The yield of the desired product was 24%.

## Claims

**1.** A process for preparing 2,2'-methylene-bis(4-hydrocarbyl-6-benzotriazolyl-phenols) having formula III: wherein:

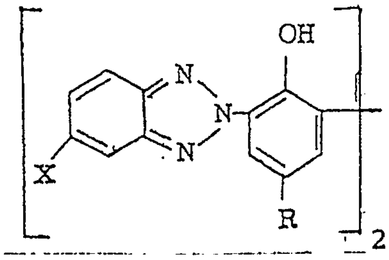

III

R is selected from the group consisting of alkyl having from one to twelve carbon atoms; arylalkyl having from seven to fourteen carbon atoms and cycloalkyl having from three to twelve carbon atoms; X is selected from the group consisting of hydrogen, halogen, alkyl having from one to twelve carbon atoms, aryl having from six to ten carbon atoms, arylalkyl having from seven to sixteen carbon atoms, alkoxy having from one to twelve carbon atoms, aryloxy having from six to ten carbon atoms; and arylalkoxy having from seven to sixteen carbon atoms;
comprising

(1) reacting a 4-hydrocarbyl-6-benzotriazolyl phenol having the formula I:

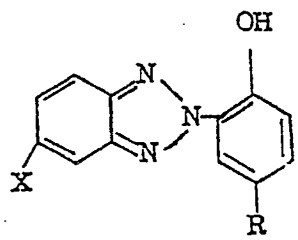

I

with an amine $HNR_1R_2$ and formaldehyde in an inert organic solvent to produce a Mannich base having formula II:

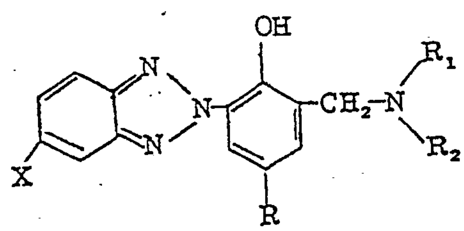

II;

$R_1$ and $R_2$ are selected from the group consisting of hydrogen; alkyl having from one to six carbon atoms, and $R_1$ and $R_2$ taken together to form a four to six member heterocyclic ring including a nitrogen atom, pro vided that at least one of $R_1$ and $R_2$ is not hydrogen; and

(2) reacting the Mannich base with itself or a 4-hydrocarbyl-6-benzotriazolyl phenol having formula I, thereby forming a 2,2'-methylene-bis-(4-hydrocarbyl-6-benzotriazolyl-phenol) of formula III.

**2.** A process according to claim 1 in which the reaction temperature in steps (1) and (2) is within the range from about 20° to about 200° C.

**3.** A process according to claim 1 in which the reaction temperature in steps (1) and (2) is within the range from about 30° to about 150° C.

**4.** A process according to one or more of the claims 1 to 3 in which an inert organic solvent that is a solvent for the reactants is used in an amount within the range from about 50 to about 500% by weight of the benzotriazolyl phenol.

**5.** A process according to one or more of the claims 1 to 4 in which the amounts of amine and formaldehyde in step (1) are within the range from about 0.5 to about 1 mole per mole of benzotriazolyl phenol.

**6.** A process according to claim 1 in which step (2) is carried out in the presence of an alkaline catalyst.

7. A process according to one or more of the claims 1 to 6, in which step (2) is carried out in the presence of an alkaline catalyst in an amount within the range from about 0.001 to about 50 parts by weight per 100 parts of Mannich base.

8. A process according to claim 1 in which step (2) is carried out in the presence of an alkaline catalyst in an amount within the range from about 0.01 to about 8 parts by weight per 100 parts of Mannich base.

9. A process according to one or more of the claims 1 to 8 in which the alkaline catalyst is a lower alkali metal alcoholate.

10. A process according to one or more of the claims 1 to 8 in which the alkaline catalyst is a lower alkali metal hydroxide.

11. A process according to one or more of the claims 1 to 8 in which the alkaline catalyst is a lower alkali metal alkaline salt.

## Revendications

1. Un procédé de préparation des 2,2'-méthytène-bis(4-hydrocarbyl-6-benzotriazolylphénols) ayant la formule III :

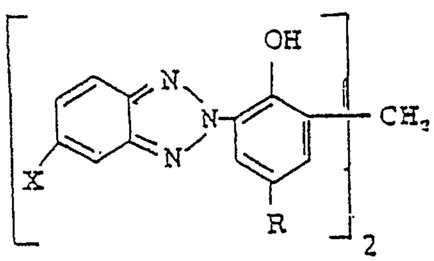

II

dans laquelle :
R est choisi dans le groupe comprenant un groupe alkyle de 1 à 12 atomes de carbone, un groupe arylalkyle de 7 à 14 atomes de carbone et un groupe cycloalkyle de 3 à 12 atomes de carbone ; X est choisi dans le groupe comprenant un atome d'hydrogène, d'halogène, un groupe alkyle de 1 à 12 atomes de carbone, un groupe aryle de 6 à 10 atomes de carbone, un groupe arylalkyle de 7 à 16 atomes de carbone, un groupe alcoxy de 1 à 12 atomes de carbone, un groupe aryloxy de 6 à 10 atomes de carbone et un groupe arylalcoxy de 7 à 16 atomes de carbone ;
ledit procédé comprenant :

(1) la réaction d'un 4-hydrocarbyl-6-benzotriazolylphénol ayant la formule I :

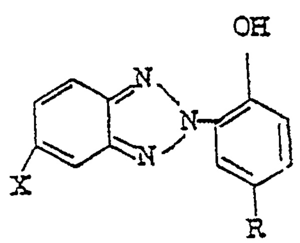

I

avec une amine $HNR_1R_2$ et avec le formaldéhyde dans un solvant organique inerte pour produire une base de Mannich ayant la formule II :

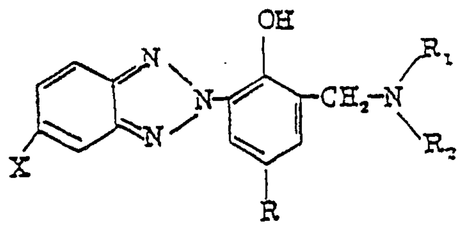

II

dans laquelle $R_1$ et $R_2$ sont choisis dans le groupe comprenant un atome d'hydrogène, un groupe alkyle de 1 à 6 atomes de carbone, et $R_1$ et $R_2$ pris ensemble forment un noyau hétérocyclique à 4 à 6 chaînons incluant un atome d'azote, à la condition qu'au moins l'un des $R_1$ et $R_2$ ne soit pas l'hydrogène ; et

(2) la réaction de la base de Mannich avec elle-même ou avec un 4-hydrocarbyl-6-benzotriazolylphénol de formule I, pour former un 2,2'-méthylène-bis-(4-hydrocarbyl-6-benzotriazolylphénol) de formule III.

2. Un procédé selon la revendication 1, selon lequel la température de réaction dans les étapes (1) et (2) est comprise dans l'intervalle d'environ 20° à environ 200° C.

3. Un procédé selon la revendication 1, selon lequel la température de réaction dans les étapes (1) et (2) est comprise dans l'intervalle d'environ 30° à environ 150° C.

4. Un procédé selon l'une ou plusieurs des revendications 1 à 3, selon lequel un solvant organique inerte qui est un solvant des réactifs est utilisé en quantité comprise dans l'intervalle d'environ 50 à environ 500 % en poids du benzotriazolylphénol.

5. Un procédé selon l'une ou plusieurs des revendications 1 à 4, selon lequel les quantités d'amine et de formaldéhyde dans l'étape (1) sont comprises dans l'intervalle d'environ 0,5 à environ 1 mol par mol de benzotriazolylphénol.

6. Un procédé selon la revendication 1, selon lequel l'étape (2) est conduite en présence d'un catalyseur alcalin.

7. Un procédé selon une ou plusieurs des revendications 1 à 6, selon lequel l'étape (2) est conduite en présence d'un catalyseur alcalin en quantité comprise dans l'intervalle d'environ 0,001 à environ 50 parties en poids pour 100 parties de base de Mannich.

8. Un procédé selon la revendication 1, selon lequel l'étape (2) est conduite en présence d'un catalyseur alcalin en quantité comprise dans l'intervalle d'environ 0,01 à environ 8 parties en poids pour 100 parties de base de Mannich.

9. Un procédé selon une ou plusieurs des revendications 1 à 8, selon lequel le catalyseur alcalin est un alcoolate de métal alcalin inférieur.

10. Un procédé selon une ou plusieurs des revendications 1 à 8, selon lequel le catalyseur alcalin est un hydroxyde de métal alcalin inférieur.

11. Un procédé selon une ou plusieurs des revendications 1 à 8, selon lequel le catalyseur alcalin est un sel alcalin de métal alcalin inférieur.

**Ansprüche**

1. Verfahren zur Herstellung von 2,2'-Methylen-bis(4-hydrocarbyl-6-benzotriazolyl-phenolen) mit der Formel III: worin

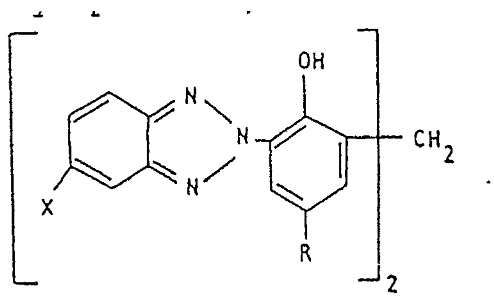

(III)

R ausgewählt ist aus der Gruppe, bestehend aus Alkyl mit 1 bis 12 Kohlenstoffatomen, Arylalkyl mit 7 bis 14 Kohlenstoffatomen und Cycloalkyl mit 3 bis 12 Kohlenstoffatomen; X ausgewählt ist aus der Gruppe, bestehend aus Wasserstoff, Halogen, Alkyl mit 1 bis 12 Kohlenstoffatomen, Aryl mit 6 bis 10 Kohlenstoffatomen, Arylalkyl mit 7 bis 16 Kohlenstoffatomen, Alkoxy mit 1 bis 12 Kohlenstoffatomen, Aryloxy mit 6 bis 10 Kohlenstoffatomen und Arylalkoxy mit 7 bis 16 Kohlenstoffatomen;
welches umfaßt:

(1) Umsetzen eines 4-Hydrocarbyl-6-benzotriazolyl-phenols der Formel I:

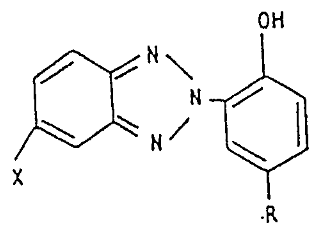

(I)

mit einem Amin $HNR_1R_2$ und Formaldehyd in einem inerten organischen Lösungsmittel zur Bildung einer Mannich-Base der Formel II
worin

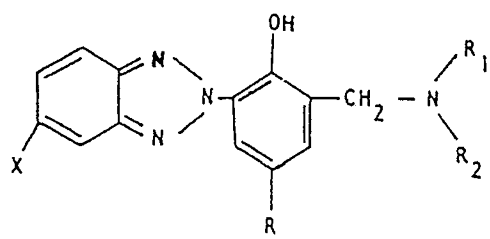

II

$R_1$ und $R_2$ ausgewählt sind aus der Gruppe, bestehend aus Wasserstoff, Alkyl mit 1 bis 6 Kohlenstoffatomen, oder $R_1$ und $R_2$ zusammen einen 4- bis 6-gliedrigen heterocyclischen Ring einschließlich eines Stickstoffatoms bilden, mit der Maßgabe, daß mindestens einer der Substituenten $R_1$ und $R_2$ eine andere Bedeutung als Wasserstoff hat, und
(2) Umsetzen der Mannich-Base mit sich selbst oder einem 4-Hydrocarbyl-6-benzotriazolyl-phenol der Formel I, um so ein 2,2'-Methylen-bis-(4-hydrocarbyl-6-benzotriazolyl-phenol) der Formel III zu bilden.

2. Verfahren nach Anspruch 1, bei welchem die Reaktionstemperatur in den Stufen (1) und (2) im Bereich von etwa 20 bis etwa 200° C liegt.

3. Verfahren nach Anspruch 1, bei welchem die Reaktionstemperatur in den Stufen (1) und (2) im Bereich von etwa 30 bis etwa 150° C liegt.

4. Verfahren nach einem oder mehreren der Ansprüche 1 bis 3, bei welchem ein inertes organischen Lösungsmittel, das ein Lösungsmittel für die Reaktionsteilnehmer ist, in einer Menge im Bereich von etwa 50 bis etwa 500 Gew.-% des Benzotriazolyl-phenols verwendet wird.

5. Verfahren nach einem oder mehreren der Ansprüche 1 bis 4, bei welchem die Mengen von Amin und

Formaldehyd in Stufe (1) im Bereich von etwa 0,5 bis etwa 1 Mol pro Mol Benzotriazolyl-phenol liegen.

6. Verfahren nach Anspruch 1, in welchem die Stufe (2) in Anwesenheit eines alkalischen Katalysators durchgeführt wird.

7. Verfahren nach einem oder mehreren der Ansprüche 1 bis 6, bei welchem die Stufe (2) in Anwesenheit eines alkalischen Katalysators in einer Menge im Bereich von etwa 0,001 bis etwa 50 Gew.-Teilen pro 100 Teile Mannich-Base durchgeführt wird.

8. Verfahren nach Anspruch 1, bei welchem die Stufe (2) in Anwesenheit eines alkalischen Katalysators in einer Menge im Bereich von etwa 0,01 bis etwa 8 Gew.-Teilen pro 100 Teile Mannich-Base durchgeführt wird.

9. Verfahren nach einem oder mehreren der Ansprüche 1 bis 8, bei welchem der alkalische Katalysator ein niederes Alkalimetallalkoholat ist.

10. Verfahren nach einem oder mehreren der Ansprüche 1 bis 8, bei welchem der alkalische Katalysator ein niederes Alkalimetallhydroxid ist.

11. Verfahren nach einem oder mehreren der Ansprüche 1 bis 8, bei welchem der alkalische Katalysator ein niederes, alkalisches Alkalimetallsalz ist.